# EUROPEAN PATENT APPLICATION

(11) **EP 1 538 143 A1**
(43) Date of publication of application: **08.06.2005**
(21) Application number: 03794296.8
(22) Date of filing: 09.09.2003
(51) Int. Cl.: C07C 263/20, C07C 265/14

(54) **LYSIN ESTER TRIISOCYANATE AND METHOD FOR PRODUCTION THEREOF**

(30) Priority: 09.09.2002 JP 2002262202
(71) Applicant: Kyowa Hakko Chemical Co., Ltd., Tokyo 103-0022 (JP)
(72) Inventor: YAMASAKI, Kenji, Yokkaichi Research Labs, Yokkaichi-shi, Mie 510-8502 (JP); IWASA, Tomohiro, Yokkaichi Research Labs, Yokkaichi-shi, Mie 510-8502 (JP); NIHONMATSU, Toshihiko,c/o Yokkaichi Research Labs., Yokkaichi-shi, Mie 510-8502 (JP); HISAMURA, Koji, c/o Yokkaichi Plant, Yokkaichi-shi, Mie 510-8502 (JP)
(74) Representative: Casalonga, Axel
(86) International application number: PCT/JP2003/011507
(87) International publication number: WO 2004/022527

(57) **Abstract**

The present invention provides a lysine ester triisocyanate represented by general formula (I): 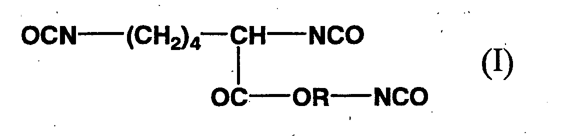 (wherein R represents lower alkylene), **characterized in that** the change in a hue value (APHA) of the lysine ester triisocyanate is 20 or less when it is stored for two weeks at 40°C in a nitrogen atmosphere under blocking light; and a process for producing the same.

## Description

### Technical Field

The present invention relates to lysine ester triisocyanates which are useful in applications such as paint and which scarcely color with time, and processes for producing the same.

### Background Art

Aliphatic isocyanates are produced by reacting their corresponding amines or salts thereof with phosgene and distilling the resulting crude isocyanates. However, in some cases, the products may color to yellow or the like. When aliphatic isocyanates are used in applications such as paint, coloration becomes greatly disadvantageous.

Methods for producing lysine ester triisocyanates having excellent hue, in which a mixture comprising the lysine ester triisocyanate is brought into contact with activated carbon and/or a metal halide prior to distillation have been reported. By such methods, lysine ester triisocyanates with a hue value (APHA) of 50 or less can be produced (e.g., refer to Japanese Unexamined Patent Application Publication No. 3462/2002). However, in all of the examples in the patent application publication described above, a mixture comprising a lysine ester triisocyanate with a high hue value is brought into contact with activated carbon and/or a metal halide at 130°C, and the lysine ester triisocyanate produced by this method undergoes degradation in hue during long-term storage and is not practically satisfactory when used in applications such as paint.

### Disclosure of Invention

An object of the present invention is to provide a lysine ester triisocyanate which scarcely colors with time and a process for producing the same.

The present invention provides the following (1) to (7).
(1) A lysine ester triisocyanate represented by general formula (I): 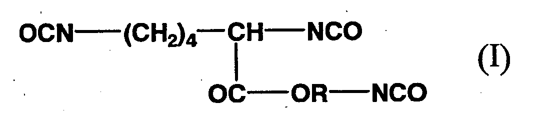 (wherein R represents lower alkylene), characterized in that the change in a hue value (APHA) of the lysine ester triisocyanate is 20 or less when it is stored for two weeks at 40°C in a nitrogen atmosphere under blocking light.
(2) The lysine ester triisocyanate according to (1), wherein R is ethylene.
(3) The lysine ester triisocyanate according to (1) or (2), wherein the hue value (APHA) before storage is 50 or less.
(4) A process for producing a lysine ester triisocyanate represented by general formula (I): 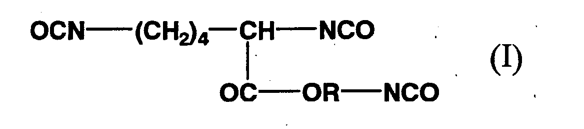 (wherein R has the same meaning as defined above), which comprises a step of bringing a mixture comprising the lysine ester triisocyanate represented by the general formula (I) into contact with activated carbon at a temperature of 10°C to 40°C.
(5) The process for producing a lysine ester triisocyanate according to (4), wherein the mixture comprising the lysine ester triisocyanate represented by general formula (I) has a hue value (APHA) of 100 or more.
(6) The process for producing a lysine ester triisocyanate according to (4) or (5), wherein the mixture comprising the lysine ester triisocyanate represented by general formula (I) is a reaction mixture obtained by reacting its corresponding triamine or a salt thereof with phosgene.
(7) The process for producing a lysine ester triisocyanate according to any one of (4) to (6), wherein after the step of bringing a mixture comprising the lysine ester triisocyanate represented by general formula (I) into contact with activated carbon, a step of subjecting the resulting mixed liquid to thin-film distillation is carried out.

Examples of the lower alkylene which is defined as the group in general formula (I) include linear or branched alkylenes having 2 to 6 carbon atoms, and specific examples thereof include ethylene, propylene, butylene, heptylene, isobutylene, pentylene, hexylene, and the like. Among these, ethylene is preferable. Hereinafter, the lysine ester triisocyanate represented by general formula (I) may be simply expressed as the lysine ester triisocyanate.

As the mixture comprising the lysine ester triisocyanate to be used as the starting material for the production process of the present invention, those having a hue value (APHA) of 100 or more are preferable, and those having APHA of 200 or more are more preferable.

The mixture comprising the lysine ester triisocyanate to be used as the starting material for the production process of the present invention may be prepared by any method. For example, a mixture comprising a lysine ester triisocyanate a hue of which hue is degraded during storage ; or a mixture comprising a lysine ester triisocyanate prepared by reacting its corresponding triamine or a salt thereof with phosgene and, as needed, by distilling the resulting reaction mixture may be used. The process for preparing the latter mixture is described below in detail.

The afore-mentioned triamine (e.g., lysine β-aminoethyl ester and the like) or a salt thereof can be produced, for example, by the process described in Japanese Published Unexamined Patent Application No.65253/1993 and the like or similar method thereto. For example, a lysine hydrochloride and an amino alcohol or a hydrochloride thereof are subjected to esterification reaction under hydrogen chloride gas flow and under reduced pressure while removing water, and then reaction mixture thereof is isolated by crystallization using an alcohol solvent, such as methanol, ethanol, propanol, isopropyl alcohol, butanol or the like to produce the triamine. Furthermore, the resulting product may be subjected to recrystallization or dehydration, if required.

Examples of the salt of the triamine include inorganic acid salts, such as hydrochlorides, sulfates, nitrates, and the like, and organic acid salts, such as p-toluenesulfonates and the like. Among these, hydrochlorides are preferable.

The reaction of the triamine or the salt thereof with phosgene is carried out, for example, by a method according to Japanese Published Examined Patent Application No. 26775/1985, in which the triamine or the salt thereof is suspended in an inert solvent, such as an aromatic hydrocarbon (e.g., benzene, toluene, o-xylene, m-xylene, p-xylene and the like), a chlorinated aromatic hydrocarbon (e.g., chlorobenzene, o-dichlorobenzene, m-dichlorobenzene, op-dichlorobenzene and the like), a chlorinated aliphatic hydrocarbon (e.g., trichloroethane and the like),' or a chlorinated alicyclic hydrocarbon (e.g., chlorocyclohexane), and phosgene is circulated in the resulting suspension preferably at a temperature in a range of 80°C to 150°C.

Phosgene is used preferably in an amount of 5 to 15 equivalents to one amino group of the triamine or the salt thereof.

The reaction liquid obtained by the reaction with phosgene may be directly used as the mixture comprising the lysine ester triisocyanate as the starting material in the production process of the present invention. Preferably, the reaction liquid is subjected to distillation (preferably, at a temperature in a range of 40°C to 150°C, and at a pressure in a range of 0.8 to 2.0 kPa) to produce the mixture comprising the lysine ester triisocyanate to be used as the starting material in the production process of the present invention.

Next, the production process of the present invention will are described below.

The moisture content of the activated carbon is preferably 30% by weight or less, and more preferably 20% by weight or less.

Amount of the activated carbon used is preferably 0.01% to 100% by weight, more preferably 0.1% to 50% by weight, and still more preferably 0.5% to 20% by weight, relative to the mixture comprising the lysine ester triisocyanate.

Although the temperature at which the mixture comprising the lysine ester triisocyanate, which is brought into contact with activated carbon, is in a range of 10°C to 40°C, it is preferable that the temperature is in a range of 10°C to 30°C.

When the mixture comprising the lysine ester triisocyanate is brought into contact with activated carbon, a solvent may be used. Example of the solvent include aromatic solvents, such as toluene xylene, and the like; ketone solvents, such as acetone, methyl ethyl ketone, methyl isobutyl ketone, cyclohexanone and the like; ester solvents, such as ethyl acetate, butyl acetate, isobutyl acetate; glycol ether ester solvents and the like, such as ethylene glycol ethyl ether acetate, propylene glycol methyl ether acetate, 3-methyl-3-methoxybutyl acetate, ethyl-3-ethoxy propionate and the like; and ether solvents, such as tetrahydrofuran, dioxane and the like. The solvent is used in an amount of preferably 0.1 to 20 times (by weight), and more preferably 0.1 to 10 times (by weight) relative to the amount of the mixture comprising the lysine ester triisocyanate.

The time of contact between the mixture comprising the lysine ester triisocyanate and activated carbon is not particularly limited, but is usually preferably in a range of 5 minutes to 10 hours. As the method of contact, either batch processing or continuous processing may be used. The contact may be performed under any pressure of normal pressure, under increased pressure, and under reduced pressure.

After the afore-mentioned contact process, the filtrate obtained by removing activated carbon by filtration is preferably further subjected to thin-film distillation. The thin-film distillation is performed preferably at a pressure of 13.3 Pa or less and at a temperature in a range of 80°C to 250°C, and more preferably at a pressure in a range of 0.000001 to 13.3 Pa and at a temperature in a range of 100°C to 180°C.

Furthermore, the step of bringing the mixture comprising the lysine ester triisocyanate into contact with activated carbon may be performed two or more times repeatedly.

As the lysine ester triisocyanate of the present invention including the one obtained by the production process of the present invention, those having has an APHA of 50 or less is preferable.

When the lysine ester triisocyanate of the present invention is stored for two weeks at 40°C in a nitrogen atmosphere under blocking light, the change in APHA is 20 or less, and preferably 10 or less.

The lysine ester triisocyanate of the present invention scarcely color with time and is useful particularly in applications such as paint.

### Best Mode for Carrying Out the Invention

The purity of the lysine diisocyanate-β-isocyanate ethyl ester (LTI) obtained in each of Example 1 and Comparative Examples 1 and 2 was analyzed using the high-performance liquid chromatography (HPLC) by the method described below.

### (Preparation of sample for HPLC analysis and method for analysis of the same)

1) About 1.0 ml of aniline is placed in a sample bottle.
2) About 50 mg of LTI is further placed in the sample bottle.
3) After about 1.5 ml of acetonitrile is added into the bottle, the bottle is left to stand for 5 minutes, and 200 mg of benzophenone as an internal standard substance is further added into the bottle.
4) A diluent solvent (ethanol/acetonitrile = 2/1 (volume ratio)) in an amount of 100 ml is added and 50 µl of 85% phosphoric acid aqueous solution is further added to form a solution.
5) The afore-mentioned sample solution (1 ml) is diluted with 5 ml of diluent solvent (ethanol/acetonitrile = 2/1 (volume ratio)) and then analyzed by HPLC.

### (HPLC analysis conditions)

Column: YMC A312 (manufactured by YMC Corporation: 6 × 150 mm)
Column temperature: 40°C
Mobile phase: acetonitrile/Methanol/0.05% phosphoric acid aqueous solution = 4/1/5 (volume ratio)
Flow rate: 2 ml/min
Detection: UV (240 nm)

The hue value (APHA) was determined by a method according to JIS K1545.

The method according to JIS K1545 is described below in detail.

A sample is poured into a colorimetric tube up to a marked line and placed on an appropriate white board. The colorimetric tube is slightly lifted from the white board and the sample is looked down to visually compare to standard solutions to select a standard solution with the most approximate to the sample color. The APHA number of the selected standard solution is considered as the hue of the sample.

A standard solution of APHA 500 is prepared as follows. About 1.25 g of potassium chloroplatinate and about 1 g of cobalt chloride are dissolved in about 100 ml of hydrochloric acid so that 0.500 g of platinum and 0.250 g of cobalt are contained, and the solution is diluted with water to 1,000 ml. A standard solution of APHA 500 is thereby prepared. In order to prepare a standard solution of APHA 10 for example, 2.0 ml of the standard solution of APHA 500 and 98.0 ml of water are mixed.

### Reference Example 1: Production of mixture comprising LTI

Lysine monohydrochloride (102 g) and ethanolamine (109 g) were mixed, and hydrogen chloride gas in an amount corresponding to 1.2 mole times ethanolamine was injected into the mixture for over 2 hours. While controlling the mixture at 120°C and at 33 kPa, hydrogen chloride gas was further circulated at 0.2 L/mol lysine monohydrochloride/min, and the resulting mixture was retained for 7 hours. After the reaction, crystallization was performed with a mixed solvent of methanol and n-butanol. Filtration was then performed and the filtrate was dried under reduced pressure at 60°C and at 66 Pa, and thereby 100 g of lysine-β-aminoethyl ester trihydrochloride was obtained.

The resulting lysine-β-aminoethyl ester trihydrochloride (100 g) was mixed with 500 g of o-dichlorobenzene, and while heating at 130°C, phosgene gas was injected into the mixture for over 12 hours at a flow rate of 3 mol/lysine-β-aminoethyl ester trihydrochloride/hour. Subsequently, nitrogen gas was injected to remove phosgene, and o-dichlorobenzene was distilled at 1.3 kPa between 50°C to 120°C. A mixture (100 g) comprising LTI with a purity of 80% was thereby obtained.

### Example 1

To 100 g of the mixture comprising the LTI with a purity of 80% obtained in Reference Example 1, activated carbon in an amount of 3 g (3.0% by weight relative to crude LTI) was added, and stirring was carried out at 25°C for 2 hours. Subsequently, solids including activated carbon were removed by filtration. To the resulting filtrate (91 g), activated carbon in an amount of 2.7 g (3.0% by weight relative to the filtrate) was added, and stirring was carried out at 25°C for 2 hours. Subsequently, solids including activated carbon were removed by filtration. The resulting filtrate was distilled at 140°C, under a pressure of 6.6 Pa using a falling-film type molecular distillation apparatus [Shibata molecular distillation apparatus (Model MS-300) manufactured by Shibata Kagaku Kikai Kogyo Co., Ltd; the same apparatus being used in the following example and comparative examples], and thereby 65 g of LTI with a purity of 99% was obtained. The hue value (APHA) of the LTI was 25.

The LTI was placed in a fully nitrogen substituted sample bottle made of glass and stored at 40°C under blocking light. The hue value (APHA) after two weeks was 30.

### Comparative Example 1

The mixture comprising the LTI with a purity of 80% obtained in Reference Example 1 (100 g), was untreated with activated carbon, and was distilled using the falling-film type molecular distillation apparatus under the same conditions as those in Example 1, and thereby 80 g of LTI with a purity of 98% was obtained. The hue value (APHA) of the LTI was 200. The LTI was placed in a fully nitrogen substituted sample bottle made of glass and stored at 40°C. under blocking light. The hue value (APHA) of the LTI after two weeks was 500.

### Comparative Example 2 (Method in the Example of Japanese Published Unexamined Patent Application No.3462/2000).

To 100 g of the mixture comprising the LTI with a purity of 80% obtained in Reference Example 1, activated carbon in an amount of 0.5 g (0.5% by weight relative to crude LTI) and 0.5 g of zinc chloride were added. Stirring was carried out at 130°C for 2 hours. Subsequently, solids including activated carbon and zinc chloride were removed by filtration. The resulting filtrate was distilled using the falling-film type molecular distillation apparatus under the same conditions as recited in Example 1, and thereby 70 g of LTI with a purity of 99% was obtained. The hue value (APHA) of the LTI was 30. The LTI was placed in a fully nitrogen substituted sample bottle made of glass and stored at 40°C under blocking light. The hue value (APHA) of the LTI after two weeks was 80.

### Industrial Applicability

In accordance with the present invention, a lysine ester triisocyanate which scarcely colors with time and a process for producing the same are provided.

## Claims

1. A lysine ester triisocyanate represented by general formula (I): 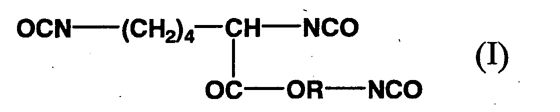 (wherein R represents lower alkylene), **characterized in that** the change in a hue value (APHA) of the lysine ester triisocyanate is 20 or less when it is stored for two weeks at 40°C in a nitrogen atmosphere under blocking light.

2. The lysine ester triisocyanate according to Claim 1, wherein R is ethylene.

3. The lysine ester triisocyanate according to Claim 1 or 2, wherein the hue value (APHA) before storage is 50 or less.

4. A process for producing a lysine ester triisocyanate represented by general formula (I): 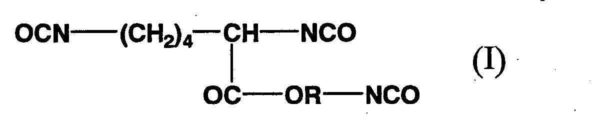 (wherein R has the same meaning as defined above), which comprises a step of bringing a mixture comprising the lysine ester triisocyanate represented by the general formula (I) into contact with activated carbon at a temperature of 10°C to 40°C.

5. The process for producing a lysine ester triisocyanate according to Claim 4, wherein the mixture comprising the lysine ester triisocyanate represented by general formula (I) has a hue value (APHA) of 100 or more.

6. The process for producing a lysine ester triisocyanate according to Claim 4 or 5, wherein the mixture comprising the lysine ester triisocyanate represented by general formula (I) is a reaction mixture obtained by reacting its corresponding triamine or a salt thereof with phosgene.

7. The process for producing a lysine ester triisocyanate according to any one of Claims 4 to 6, wherein after the step of bringing a mixture comprising the lysine ester triisocyanate represented by general formula (I) into contact with activated carbon, a step of subjecting the resulting mixed liquid to thin-film distillation is carried out.
